(19) Europäisches Patentamt
European Patent Office
Office européen des brevets

(11) EP 1 533 619 B1

(12) **EUROPEAN PATENT SPECIFICATION**

(45) Date of publication and mention of the grant of the patent:
**29.04.2009 Bulletin 2009/18**

(51) Int Cl.:
***G01N 33/68*** *(2006.01)*

(21) Application number: **04026956.5**

(22) Date of filing: **12.11.2004**

(54) **Specific markers for metabolic syndrome**

Spezifische Marker für Stoffwechselssyndrome

Marqueurs spécifiques du syndrome métabolique.

(84) Designated Contracting States:
**CH DE ES FR GB IT LI**
Designated Extension States:
**LT LV**

(30) Priority: **20.11.2003 US 523845 P**

(43) Date of publication of application:
**25.05.2005 Bulletin 2005/21**

(60) Divisional application:
**09151175.8 / 2 053 409**

(73) Proprietor: **F. Hoffmann-La Roche AG**
**4070 Basel (CH)**

(72) Inventors:
- **Kochan, Jarema Peter**
  **Verona, New Jersey 07044 (US)**
- **Rosinski, James Andrew**
  **Nutley, New Jersey 07110 (US)**

(56) References cited:
**WO-A-03/021229**
**DE-A1- 3 737 367**
**DE-A1- 3 810 331**

- **GABRIELSSON BRITT G ET AL: "High expression of complement components in omental adipose tissue in obese men." OBESITY RESEARCH. JUN 2003, vol. 11, no. 6, June 2003 (2003-06), pages 699-708, XP002318885 ISSN: 1071-7323**
- **WEBER-HAMANN BETTINA ET AL: "Hypercortisolemic depression is associated with increased intra-abdominal fat" PSYCHOSOMATIC MEDICINE, vol. 64, no. 2, March 2002 (2002-03), pages 274-277, XP002318886 ISSN: 0033-3174**
- **BROCHU MARTIN ET AL: "Visceral adipose tissue is an independent correlate of glucose disposal in older obese postmenopausal women" JOURNAL OF CLINICAL ENDOCRINOLOGY AND METABOLISM, vol. 85, no. 7, July 2000 (2000-07), pages 2378-2384, XP002318887 ISSN: 0021-972X**
- **KATZMARZYK PETER T ET AL: "Genetics of abdominal visceral fat levels" AMERICAN JOURNAL OF HUMAN BIOLOGY, vol. 11, no. 2, 1999, pages 225-235, XP008043512 ISSN: 1042-0533**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 533 619 B1

## Description

In both men and women, visceral adipose tissue accumulation is associated with an increased risk of the development of non-insulin dependent diabetes, myocardial infarction, stroke and other arteriosclerotic diseases and their associated risk factors, including insulin resistance, elevated blood lipids, glucose and hypertension. The clustering of these risk factors has been designated 'Metabolic Syndrome', also called 'Syndrome X', the 'Insulin Resistance Syndrome' or the 'Deadly Quartet'. This syndrome is also characterized by one or more endocrine disturbances and is therefore also called 'Neuro-endocrine Syndrome' (Marin, P. Neuroendocrine News, 21(3) 1996, 2). These disturbances include low serum levels of sex steroids (testosterone in men, and estrogens in women), signs of a decreased action of growth hormone, and an excessive secretion of cortisol. The latter has been shown clinically as a major causative process for the development of Metabolic Syndrome as demonstrated by successful treatment with the cortisol synthesis inhibitor ketoconazole (WO 96/04912).

Conditions related to Metabolic Syndrome include diabetes mellitus type II (IDDM), non-insulin dependent diabetes (NIDDM), myocardial infarction, stroke and other arteriosclerotic diseases as well as the risk factors for these diseases, insulin resistance in general, abdominal obesity caused by accumulation of visceral adipose tissue, elevated serum lipids, and raised diastolic and/or systolic blood pressure.

Visceral adipose tissue is known as the intra-abdominal fat, the adipose depot associated with central obesity. This adipose depot is to be distinguished from the subcutaneous adipose depot, which is located throughout the body. It is the visceral adipose tissue, which has been associated with an increased risk for disorders, as well as mortality. Visceral adipose tissue plays a key role in this process by modulating whole body metabolism, in as yet undefined ways. In obesity, the relative amounts of visceral adipose tissue can vary from individual to individual, and the only means of precisely defining the levels of visceral adipose tissue is via the use of magnetic resonance imaging and by computed tomography. These complex techniques can provide a detailed determination of the levels of visceral adipose tissue, but are not available to the routine access to measure the community at large for healthcare purposes. In addition, the costs associated with MRI and CT scans are quite large, and thus not applicable to routine screening.

Peter T. Katzmarzyk et al. (American Journal of Human Biology 11:225-235 (1999)) review the recent scientific publications on the topic abdominal visceral fat (AVF) as risk factor for cardiovascular diseases or diabetes. There is evidence suggesting a genetic component to the observed variation in AVF. The glucocorticoid receptor (GRL), β3 adrenergic receptor (ADRB3) and fattiy acid binding protein 2 (FABP2) genes have been significantly associated with AVF or intra-abdominal fat levels in humans.

Bettina Weber-Hamann et al. (Psychosomatic Medicine 64:274-277 (2002)) found that hypercortisolemic depressed patients suffer from resistance to insulin and increrased visceral fat. The fact that hypercortisolemia reverses depression-related fat loss, particularly in the visceral area, might partially explain why major depression can be considered a risk factor for cardiovascular disorders.

Martin Brochu et al (The Journal of Clinical Endocrinology & Metabolism, Vol. 85, No. 7, 2000) examined the relationship between body fat distribution and glucose disposal using criterion methods for body composition, body fat distribution and physical activity levels in a cohort of older obese postmenopausal women. Results showed that obese women with high visceral adipose tissue levels had a lower glucose disposal per kg lean body mass compared to those with low visceral adipose tissue levels.

As can be seen, there is a need for a relatively simple and cost-efficient technique for measuring, monitoring and tracking levels of visceral adipose tissue as a method for diagnosing and possibly treating metabolic syndrome as well as a method for finding potential compounds for the treatment of metabolic syndrome.

According to one aspect of the present invention, a method for the measurement of levels of visceral adipose tissue comprises detecting or measuring the level of a polypeptide marker in a biological sample, the polypeptide marker comprising a polypeptide of SEQ ID No. 2.

According to another aspect of the present invention, a method for measuring the level of visceral adipose tissue in a subject comprises detecting or measuring the level of a marker in a biological sample, the nucleic acid marker comprising a nucleic acid molecule of SEQ ID No. 1.

According to yet another aspect of the present invention, there is provided a screening method for identifying a compound which is an agonist or an antagonist of a polypeptide that is predominately expressed in visceral adipose tissue, the polypeptide being the polypeptide of SEQ ID No. 2, comprising contacting said polypeptide with a compound under conditions which allow interaction of the compound with said polypeptide; determining a first level of activity of the polypeptide; determining a second level of activity of the polypeptide expressed in a host which has not been contacted with the compound; and quantitatively relating the first level of activity with the second level of activity, wherein when the first level of activity is less than the second level of activity, the compound is identified as an antagonist of the polypeptide.

According to still a further aspect of the present invention, there is provided a screening method for identifying a compound which is an inhibitor of the expression of a polypeptide that is predominately expressed in visceral adipose tissue, the polypeptide being the polypeptide of SEQ ID No. 2, comprising contacting a host which expresses the polypeptide with a compound; determining a first expression level or activity of the polypeptide; determining a second expression level or activity of the polypeptide in a host which has not been contacted with the compound; and quantitatively relating the first expression level or activity with the second expression level or activity, wherein when the first expression level or activity is less than the second expression level or activity, the compound is identified as an inhibitor of the expression of the polypeptide.

According to another aspect of the present invention, there is provided a method of correlating protein levels in a mammal with a diagnosis of the level of visceral adipose tissue, comprising determining the level of a protein comprising SEQ. ID. No. 2 in a biological sample in the mammal; and generating an index number, Y, which indicates a base level of visceral adipose tissue.

The above, and other objects, features and advantages of the present invention will become apparent from the following description read in conjunction with the accompanying drawings and claims. Figure 1 is a graph of the scaled intensity vs. log of the insulin resistance for Annexin A8 adipose levels of RNA measured by Affymetrix analysis in visceral and subcutaneous adipose tissues.

The problem of identifying genes and polypeptides suitable as markers of metabolic syndrome for early diagnosis of the disease, and the long felt need for such markers, was overcome by the present invention. It was surprisingly found that a specific gene is more selectively secreted in visceral adipose tissue. The differentially expressed gene, and the polypeptide it encodes, along with their accession numbers, are listed in Table 1.

Table 1: Visceral Adipose Secreted Protein

| Name | Abbreviation | Alias | GenBank | Locus Link | MRNA | Protein |
|---|---|---|---|---|---|---|
| Annexin A8 | ANX8 | Annexin VII, annexin VIII | X16662 SEQ ID No. 1 | 244 | NM001630 | NP001621 SEQ ID No. 2 |

The present invention provides a marker for measuring the relative amount of visceral adipose tissue present in a subject. This measurement may then be correlated to the diagnosis of metabolic syndrome or an early stage of metabolic syndrome. The marker comprises at least a polypeptide comprising SEQ ID No: 2. Thus, the term "marker" as used herein refers to one or more polypeptides that are predominately expressed in visceral adipose tissue and that can be used to measure the amount of visceral adipose tissue, and therefore, can be used to diagnose metabolic syndrome, a pre-metabolic syndromatic state or a susceptibility to develop metabolic syndrome. The markers may be used either alone or as combinations of multiple polypeptides that are known to be expressed in visceral adipose tissues.

The term "polypeptide" as used herein, refers to a polymer of amino acids, and not to a specific length. Thus, peptides, oligopeptides and proteins are included within the definition of polypeptide.

With the identification of a polypeptide predominately expressed invisceral adipose tissue, the present invention provides an in vitro method for the measurement of the levels of visceral derived secreted protein in an individual. The amounts of the measured protein can be extrapolated to the total amount of visceral adipose tissue in the individual, thereby determining the levels of visceral adipose tissue in an individual. Moreover, it will also be possible to determine whether the visceral adipose tissue differs between individuals, by measuring the levels of visceral derived secreted protein. Furthermore, differences in secreted protein can be correlated with different co-morbidities found in different individuals.

The term "differentially expressed" or "predominately expressed" in accordance with this invention relates to marker genes which express proteins that are secreted mainly by tissues and/or cells derived from visceral adipose tissue.

In accordance with the present invention, the term "biological sample" as employed herein means a sample which comprises material wherein the differential expression of marker genes may be measured and may be obtained from an individual. Particular preferred samples comprise body fluids, like blood, serum, plasma, urine, synovial fluid, spinal fluid, cerebrospinal fluid, semen or lymph, as well as body tissues, such as visceral adipose tissue.

The detection and/or measurement of the differentially expressed marker genes may comprise the detection of an increase, decrease and/or the absence of a specific nucleic acid molecule, for example RNA or cDNA, the measurement/detection of a expressed polypeptide/protein as well as the measurement/detection of a (biological) activity (or lack thereof) of the expressed protein/polypeptide. The (biological) activity may comprise enzymatic activities, activities relating to signaling pathway-events e.g. antigen-recognition as well as effector-events.

Methods for the detection/measurement of RNA and/or cDNA levels are well known in the art and comprise methods as described in the appended examples. Such methods include, but are not limited to PCR-technology, northern blots, affymetrix chips, and the like.

The term "detection" as used herein refers to the qualitative determination of the absence or presence of polypeptides. The term "measured" as used herein refers to the quantitative determination of the differences in expression of polypeptides in biological samples from patients. Additionally, the term "measured" may also refer to the quantitative determination of the differences in expression of polypeptides in biological samples from visceral adipose tissues.

Methods for detection and/or measurement of polypeptides in biological samples are well known in the art and include, but are not limited to, Western-blotting, ELISAs or RIAs, or various proteomics techniques. Monoclonal or polyclonal antibodies recognizing the polypeptide listed in Table 1, or peptide fragments thereof, can either be generated for the purpose of detecting the polypeptides or peptide fragments, eg. by immunizing rabbits with purified proteins, or known antibodies recognizing the polypeptides or peptide fragments can be used. For example, an antibody capable of binding to the denatured proteins, such as a polyclonal antibody, can be used to detect the peptides of this invention in a Western Blot. An example for a method to measure a marker is an ELISA. This type of protein quantitation is based on an antibody capable of capturing a specific antigen, and a second antibody capable of detecting the captured antigen. A further method for the detection of a diagnostic marker for the measurement of levels of visceral adipose tissue is by analyzing biopsy specimens for the presence or absence of the markers of this invention. Methods for the detection of these markers are well known in the art and include, but are not limited to, immunohistochemistry or immunofluorescent detection of the presence or absence of the polypeptides of the marker of this invention. Methods for preparation and use of antibodies, and the assays mentioned hereinbefore are described in Harlow, E. and Lane, D. Antibodies: A Laboratory Manual, (1988), Cold Spring Harbor Laboratory Press.

For diagnosis of visceral adipose tissue levels, suitable biological samples need to be analyzed for the presence or absence of a marker. The biological samples can be serum, plasma, or various tissues including cells of adipose tissue. Cells from adipose tissue can be obtained by any known method, such as ERCP, secretin stimulation, fine-needle aspiration, cytologic brushings and large-bore needle biopsy.

It is also possible to diagnose visceral adipose tissue levels by detecting and/or measuring nucleic acid molecules coding for the marker hereinbefore described. Preferably, the nucleic acid molecule is RNA or DNA.

In one embodiment of the present invention, the in vitro method herein before described comprises comparing the expression levels of at least one of the nucleic acids encoding the polypeptide marker in an individual known to have elevated levels of visceral adipose tissue, to the expression levels of the same nucleic acids in an individual known to have low or normal levels of visceral adipose tissue.

In another embodiment of the present invention the in vitro method herein before described comprises comparing the expression level of the marker in an individual known to have elevated levels of visceral adipose tissue, to the expression levels of the same nucleic acids in an individual known to have low or normal levels of visceral adipose tissue. In a more preferred embodiment of the in vitro method, an increase of the expression levels of the marker is indicative of the susceptibility to develop metabolic syndrome.

Yet, in another embodiment of the present invention, the inventive in vitro method comprises a method, wherein the detection and/or measuring step is carried out by detecting and/or measuring protein(s)/polypeptide(s) or a fragment thereof encoded by the gene having SEQ. ID. No. 1. Again, these detection/measuring steps comprise methods known in the art, like inter alia, proteomics, immuno-chemical methods like Western-blots, ELISAs and the like.

For polypeptides that are associated with the cell membrane on the cell surface, or which are expressed as transmembrane or integral membrane polypeptides, the interaction of a compound with the polypeptides can be detected with different methods which include, but are not limited to, methods using cells that either normally express the polypeptide or in which the polypeptide is overexpressed, eg. by detecting displacement of a known ligand which is labeled by the compound to be screened. Alternatively, membrane preparations may be used to test for interaction of a compound with such a polypeptide.

Interaction assays to be employed in the method disclosed herein may comprise FRET-assays (fluorescence resonance energy transfer; as described, inter alia, in Ng, Science 283 (1999), 2085-2089 or Ubarretxena-Belandia, Biochem. 38 (1999), 7398-7405), TR-FRETs and biochemical assays as disclosed herein. Furthermore, commercial assays like "Amplified Luminescent Proximity Homogenous Assay™" (BioSignal Packard) may be employed. Further methods are well known in the art and, inter alia, described in Fernandez, Curr. Opin. Chem. Biol. 2 (1998), 547-603.

The "test for interaction" may also be carried out by specific immunological and/or biochemical assays which are well known in the art and which comprise, e.g., homogenous and heterogenous assays as described herein below. The interaction assays employing read-out systems are well known in the art and comprise, inter alia, two-hybrid screenings (as, described, inter alia, in EP-0 963 376, WO 98/25947, WO 00/02911; and as exemplified in the appended examples), GST-pull-down columns, coprecipitation assays from cell extracts as described, inter alia, in Kasus-Jacobi, Oncogene 19 (2000), 2052-2059, "interaction-trap" systems (as described, inter alia, in US 6,004,746) expression cloning (e.g. lamda gt11), phage display (as described, inter alia, in US 5,541,109), in vitro binding assays and the like. Further interaction assay methods and corresponding read out systems are, inter alia, described in US 5,525,490, WO 99/51741, WO 00/17221, WO 00/14271 or WO 00/05410. Vidal and Legrain (1999) in Nucleic Acids Research 27, 919-929 describe, review and summarize further interaction assays known in the art which may be employed in accordance with the present invention.

Homogeneous (interaction) assays comprise assays wherein the binding partners remain in solution and comprise assays, like agglutination assays. Heterogeneous assays comprise assays like, inter alia, immuno assays, for example, Enzyme Linked Immunosorbent Assays (ELISA), Radioactive Immunoassays (RIA), Immuno Radiometric Assays (IRMA), Flow Injection Analysis (FIA), Flow Activated Cell Sorting (FACS), Chemiluminescent Immuno Assays (CLIA) or Electrogenerated Chemiluminescent (ECL) reporting.

The present invention further provides a screening method for identifying and/or obtaining a compound which is an agonist or an antagonist of a polypeptide comprising a polypeptide having SEQ. ID. No. 2 that is predominantly expressed in visceral adipose tissue, comprising the steps of a) contacting the polypeptide with a compound identified and/or obtained by the screening method described above under conditions which allow interaction of the compound with the polypeptide; b) determining the activity of the polypeptide; c) determining the activity of the polypeptide expressed in the host as defined in (a), which has not been contacted with the compound; and d) quantitatively relating the activity as determined in (b) and (c), wherein a decreased activity determined in (b) in comparison to (c) is indicative for an agonist or antagonist. This screening assay can be performed either as an in vitro assay, or as a host-based assay. The host to be employed in the screening methods of the present invention and comprising and/or expressing a polypeptide having SEQ. ID. No. 2 may comprise prokaryotic as well as eukaryotic cells. The cells may comprise bacterial cells, yeast cells, as well as cultured (tissue) cell lines, inter alia, derived from mammals. Furthermore animals may also be employed as hosts, for example a non-human transgenic animal. Accordingly, the host (cell) may be transfected or transformed with the vector comprising a nucleic acid molecule coding for a polypeptide having SEQ. ID. No. 2. The host cell or host may therefore be genetically modified with a nucleic acid molecule encoding such a polypeptide or with a vector comprising such a nucleic acid molecule. The term "genetically modified" means that the host cell or host comprises in addition to its natural genome a nucleic acid molecule or vector coding for a polypeptide having SEQ. ID. No. 2 or at least a fragment thereof. The additional genetic material may be introduced into the host (cell) or into one of its predecessors/parents. The nucleic acid molecule or vector may be present in the genetically modified host cell or host either as an independent molecule outside the genome, preferably as a molecule which is capable of replication, or it may be stably integrated into the genome of the host cell or host.

As mentioned herein above, the host cell of the present invention may be any prokaryotic or eukaryotic cell. Suitable prokaryotic cells are those generally used for cloning like E. coli or Bacillus subtilis. Yet, these prokaryotic host cells are also envisaged in the screening methods disclosed herein. Furthermore, eukaryotic cells comprise, for example, fungal or animal cells. Examples for suitable fungal cells are yeast cells, preferably those of the genus Saccharomyces and most preferably those of the species Saccharomyces cerevisiae. Suitable animal cells are, for instance, insect cells, vertebrate cells, preferably mammalian cells, such as e.g. CHO, HeLa, NIH3T3 or MOLT-4. Further suitable cell lines known in the art are obtainable from cell line depositories, like the American Type Culture Collection (ATCC).

A compound which interacts with a polypeptide listed in table 1 and which inhibits or antagonizes the polypeptide is identified by determining the activity of the polypeptide in the presence of the compound.

The term "activity" as used herein relates to the functional property or properties of a specific polypeptide. For the enzymes, the term "activity" relates to the enzymatic activity of a specific polypeptide. For adhesion molecules, the term "activity" relates to the adhesive properties of a polypeptide and may be determined using assays such as, but not limited to, adhesion assays, cell spreading assays, or in vitro interaction of the adhesion molecule with a known ligand. For cytoskeletal proteins, the term "activity" relates to the regulation of the cytoskeleton by such polypeptides, or to their incorporation into the cytoskeleton. As a non-limiting example, the ability of Gelsolin to regulate actin polymerization, or of Filamin A to promote orthogonal branching of actin filaments, may be determined using in vitro actin polymerization assays. Activity in relation to the regulation of cytoskeletal structures may further be determined by, as non-limiting examples, cell spreading assays, cell migration assays, cell proliferation assays or immunofluorescence assays, or by staining actin filaments with fluorescently labeled phalloidin. For ion channels the term "activity" relates to ion flux (Chloride lux) across the membrane. For transcription factors, the term "activity" relates to their ability to regulate gene transcription. The transcriptional activity of a gene can be determined using commonly used assays, such as a reporter gene assay. For growth factors and hormones or their receptors, the term "activity" relates to their ability to bind to their receptors or ligands, respectively, and to induce receptor activation and subsequent signaling cascades, and/or it relates to the factor's or receptor's ability to mediate the cellular function or functions eventually caused by growth factor or hormone mediated receptor activation. Growth factor or hormone binding to receptors can be determined by commonly known ligand binding assays. Receptor activation can be determined by testing for receptor autophosphorylation, or by assaying for modification or recruitment of downstream signaling mediators to the receptors (by immunoprecipitation and Western Blotting of signaling complexes). Cellular functions regulated by growth factors or hormones and their receptors can be cell proliferation (eg determined by using thymidine incorporation or cell counts), cell migration assays (eg determined by using modified Boyden chambers), cell survival or apoptosis assays (eg determined by using DAPI staining), angiogenesis assays (eg in vitro assays to measure endothelial tube formation that are commercially available). In addition to these assays, other assays may be used as well to determine these and other cellular functions.

Inhibitors, antagonists, activators or agonists as identified and/or obtained by the methods of the present invention are particularly useful in the therapeutic management, prevention and or treatment of metabolic syndrome related comorbidities.

Inhibitors or antagonists of a polypeptide having SEQ. ID. No. 2 may be identified by the screening method described above when there is a decreased activity determined in the presence of the compound in comparison to the absence of the compound in the screening method, which is indicative for an inhibitor or antagonist.

Therefore, potential inhibitors or antagonists to be identified, screened for and/or obtained with the method of the present invention include molecules, preferably small molecules which bind to, interfere with and/or occupy relevant sites on the expressed marker genes that are predominately present in visceral adipose tissue.

It is furthermore envisaged that such inhibitors interfere with the synthesis/production of (functional) upregulated marker genes or gene products, like, e.g. anti-sense constructs, ribozymes and the like. The inhibitors and/or antagonist which can be screened for and obtained in accordance with the method of the present invention include, inter alia, peptides, proteins, nucleic acids including DNA, RNA, RNAi, PNA, ribozymes, antibodies, small organic compounds, small molecules, ligands, and the like.

Accordingly, the inhibitor and/or antagonist of differentially expressed marker genes may comprises (an) antibody(ies). The antibody(ies) may comprise monoclonal antibodies as well as polyclonal antibodies. Furthermore, chimeric antibodies, synthetic antibodies as well as antibody fragments (like Fab, F(ab)2, Fv, scFV), or a chemically modified derivative of antibodies are envisaged. It is envisaged that the antibodies bind to the marker gene or its gene product and/or interfere its activity.

In addition, oligonucleotides and/or aptamers which specifically bind to the marker genes as defined herein or which interfere with the activity of the marker genes are envisaged as inhibitors and/or antagonists. The term "oligonucleotide" as used in accordance with the present invention comprises coding and non-coding sequences, it comprises DNA and RNA and/or comprises also any feasible derivative. The term "oligonucleotide" further comprises peptide nucleic acids (PNAs) containing DNA analogs with amide backbone linkages (Nielson, Science 274 (1991), 1497-1500). Oligonucleotides which may inhibit and/or antagonize the marker gene activity and which can be identified and/or obtained by the method of the present invention can be, inter alia, easily chemically synthesized using synthesizers which are well known in the art and are commercially available like, e.g., the AB1 394 DNA-RNA Synthesizers. Additionally, the use of synthetic small interfering dsRNAs of ~22 nt (siRNAs) may be used for suppressing gene expression.

Further to the screening methods disclosed above, this invention provides An ex vivo screening method for identifying and/or obtaining a compound which is an inhibitor of the expression of a polypeptide having SEQ. ID. No. 2 that is predominately expressed in visceral adipose tissue, comprising the steps of a) contacting a host which expresses the polypeptide with a compound; b) determining the expression level and/or activity of the polypeptide; c) determining the expression level and/or activity of the polypeptide in the host as defined in (a), which has not been contacted with the compound; and d) quantitatively relating the expression level of the polypeptide as determined in (b) and (c), wherein a decreased expression level determined in (b) in comparison to (c) is indicative for an inhibitor of the expression of the polypeptide.

An inhibitor of the expression of a polypeptide having SEQ. ID. No. 2 is identified by the screening method described hereinbefore when a decreased expression of the protein is determined in the presence of the compound in comparison to the absence of the compound in the screening method, which is indicative for an inhibitor of expression of a polypeptide.

The term "express" as used herein relates to expression levels of a polypeptide listed in table 1 that is predominately expressed in visceral adipose tissue. Preferably, expression levels are at least 2 fold, more preferably at least 3 fold, even more preferably at least 4 fold, most preferably at least 5 fold higher in visceral adipose tissue cells than in, for example, subcutaneous adipose tissue.

Within the scope of the present invention, antibodies against the protein comprising SEQ. ID. No. 2, or antigen-binding fragments thereof, may be used in an in vitro method for the measurement of levels of visceral adipose tissue.

As mentioned herein above, the inhibitor and/or antagonist may also comprise small molecules. Small molecules, however may also be identified as activators or agonists by the herein disclosed methods. The term "small molecule" relates, but is not limited to small peptides, inorganic and/or organic substances or peptide-like molecules, like peptide-analogs comprising D-amino acids.

Furthermore, peptidomimetics and/or computer aided design of appropriate antagonist, inhibitors, agonists or activators may be employed in order to obtain candidate compounds to be tested in the inventive method. Appropriate computer systems for the computer aided design of, e.g., proteins and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used in combination with the method of the invention for, e.g., optimizing known compounds, substances or molecules. Appropriate compounds can also be identified by the synthesis of peptidomimetic combinatorial libraries through successive chemical modification and testing the resulting compounds, e.g., according to the methods described herein. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, the three-dimensional and/or crystallographic structure of inhibitors activators, agonists or activators of the markers of the present invention or of the nucleic acid molecule encoding the expressed markers can be used for the design of peptidomimetic inhibitors, antagonists, agonists or activators to be tested in the method of the invention (Rose, Biochemistry 35 (1996), 12933-12944; Rutenber, Bioorg. Med. Chem. 4 (1996), 1545-1558).

The compounds to be screened with the method(s) of the present invention do not only comprise single, isolated compounds. It is also envisaged that mixtures of compounds are screened with the method of the present invention. It is also possible to employ extracts, like, inter alia, cellular extracts from prokaryotic or eukaryotic cells or organisms.

In addition, the compound identified or refined by the inventive method can be employed as a lead compound to achieve, modified site of action, spectrum of activity, organ specificity, and/or improved potency, and/or decreased toxicity (improved therapeutic index), and/or decreased side effects, and/or modified onset of therapeutic action, duration of effect, and/or modified pharmakinetic parameters (resorption, distribution, metabolism and excretion), and/or modified physico-chemical parameters (solubility, hygroscopicity, color, taste, odor, stability, state), and/or improved general specificity, organ/tissue specificity, and/or optimized application form and route may be modified by esterification of carboxyl groups, or esterification of hydroxyl groups with carbon acids, or esterification of hydroxyl groups to, e.g. phosphates, pyrophosphates or sulfates or hemi succinates, or formation of pharmaceutically acceptable salts, or formation of pharmaceutically acceptable complexes, or synthesis of pharmacologically active polymers, or introduction of hydrophylic moieties, or introduction/exchange of substituents on aromates or side chains, change of substituent pattern, or modification by introduction of isosteric or bioisosteric moieties, or synthesis of homologous compounds, or introduction of branched side chains, or conversion of alkyl substituents to cyclic analogues, or dramatization of hydroxyl group to ketales, acetales, or N-acetylation to amides, phenylcarbamates, or synthesis of Mannich bases, imines, or transformation of ketones or aldehydes to Schiffs bases, oximes, acetales, ketales, enolesters, oxazolidines, thiozolidines or combinations thereof.

Additionally, a compound or a plurality of compounds which is obtainable by the method disclosed herein can be used for the preparation of a diagnostic composition for diagnosing the level of visceral adipose tissue in a patient. It is, for example envisaged that specific antibodies, fragments thereof or derivatives thereof which specifically detect or recognize differentially expressed marker gene products as disclosed herein be employed in such diagnostic compositions. Yet, specific primers/primer pairs which may detect and/or amplify the marker gene of the present invention may be employed in the diagnostic compositions.

Accordingly, the compound to be used in the pharmaceutical as well as in the diagnostic composition may comprises an antibody, an antibody-derivative, an antibody fragment, a peptide or a nucleic acid, like primers/primer pairs as well as anti-sense constructs, RNAi or ribozymes.

The diagnostic composition may also comprise suitable means for detection known in the art.

The present invention also provides the use of a nucleic acid of SEQ ID No. 1 as a marker in the diagnosis of visceral adipose tissue accumulation; and the use of a polypeptide of SEQ ID No. 2, as a marker for the diagnosis of visceral adipose tissue accumulation.

The invention is further described by reference to the following biological examples which are merely illustrative and are not to be construed as a limitation of scope.

Examples:

Total RNA was extracted using Ultraspec® RNA (Biotecx, Houston, TX) according to the manufacturer's protocol, and purified using the RNeasy Mini kit (Qiagen, Valencia, CA) with DNase treatment. Double-stranded cDNA was synthesized from 10 ug total RNA by SuperScript™ Double-Stranded cDNA Synthesis Kit (Life Technology, Rock-ville, MD) using the T7-T24 primer. The double-stranded cDNA product was purified by phenol/chloroform/isoamyl extraction using phase lock gels (Eppendorf, Westbury, NY). Double-stranded cDNA was further converted into cRNA using the *in vitro* transcription (IVT) MEGAscript™ T7 kit (Ambion, Austin, TX) and labelled with biotinylated nucleotides[1]. The *in vitro* transcription product was purified using the RNeasy Mini kit (Qiagen, Valencia, CA), and fragmented as described (Wodicka L, Dong H, Mittmann M, Ho MH, Lockhart DJ. Genome-wide expression monitoring in Saccharomyces cerevisiae. Nat Biotechnol 1997;15:1359-67). Hybridization of the fragmented *in vitro* transcription product to the Human Genome U95 (HG-U95) Genechip® array set was performed as suggested by the manufacturer (Affymetrix, Santa Clara, CA).

Statistical Methods

All numeric analyses were conducted on signal intensities as reported by the Affymetrix's MAS algorithms (Affymetrix Technical Note: New Statistical Algorithms for Monitoring Gene Expression on GeneChip® Probe Arrays. (2001)). Chips were each standardized to the overall mean of the all of the chips in the experiment. Genes were not separately standardized.

The analysis of the data was constructed as a linear model (Draper N., Smith H. Applied Regression Analysis, Second Edition John Wiley and Sons. New York, New York. (1966); Searle S. R. Linear Models John Wiley and Sons. New York, New York. (1971)) with factors for BMI, tissue of origin (subcutaneous vs. visceral adipose), insulin resistance (measured by HOMA), fasting glucose, fasting insulin and the interactions between tissue of origin and fasting glucose, fasting insulin, and insulin resistance respectively. Calculations were done using SAS version 8.1. The equation for the model is as follows:

$$\text{Signal Intensity} = \text{BMI} + \text{tissue} + \text{IR} + \text{glucose} + \text{insulin} + \text{tissue*IR} + \text{tissue*gluocose} + \text{tissue*insulin} + \text{error}$$

Nine statistical tests (contrasts) were then performed using this model. 1) Effect in visceral adipose; 2) Effect in subcutaneous adipose; 3) Differential effect between visceral and subcutaneous adipose. Each of those three tests was performed with the three interaction terms resulting in the final 9 tests.

Results of the model calculations and statistical contrasts were then filtered to result in the final genes of interest. Significance was defined as a p-value for the entire model less than 0.001 and a p-value for the specific contrast of less than 0.01. The p-value cutoffs were chosen so as to control for false positives while still finding the majority of true positives (Sokal R. R., Rohlf F. J. Biometry W. H. Freeman and Company. New York, New York. (1969)).

Finally genes were annotated through linking the Genbank accession numbers provided by Affymetrix with the Unigene http://www.ncbi.nlm.nih.gov /entrez/query.fcgi?db=unigene) and LocusLink (http://www.ncbi.nlm.nih.gov /LocusLink/) annotations for those accession numbers.

All references discussed throughout the above specification are herein incorporated in their entirety by reference for the subject matter they contain.

It should be understood, of course, that the foregoing relates to preferred embodiments of the invention and that modifications may be made without departing from the spirit and scope of the invention as set forth in the following claims.

## Claims

1. A method for the measurement of levels of visceral adipose tissue comprising:

   detecting or measuring the level of a polypeptide marker in a biological sample, wherein said polypeptide marker is a polypeptide comprising of SEQ ID No. 2.

2. The method of claim 1, wherein said biological sample is derived from the group consisting of serum, plasma, and cells of visceral adipose tissue.

3. The method of claim 1 or 2, wherein the level of said polypeptide marker in an individual known to have elevated levels of visceral adipose tissue is compared to the expression levels of the same polypeptide marker in an individual known to have low or normal levels of visceral adipose tissue.

**4.** The methods of any one of claims 1 to 3, wherein an increase of the level of said polypeptide marker over time is indicative of metabolic syndrome or the susceptibility to metabolic syndrome.

**5.** A method for measuring the level of visceral adipose tissue in a subject comprising:

detecting or measuring the level of a nucleic acid marker in a biological sample wherein said nucleic acid marker comprises a nucleic acid molecule of SEQ ID No. 1.

**6.** The method of claim 5, wherein said nucleic acid marker is RNA.

**7.** The method claim 5 or 6, wherein the expression level of said nucleic acid marker in an individual known to have elevated levels of visceral adipose tissue is compared to the expression levels of the same nucleic acid marker in an individual known to have low or normal levels of visceral adipose tissue.

**8.** The method of any one of claims 5 to 7, wherein an increase of the expression level of said nucleic acid marker over time is indicative of metabolic syndrome or the susceptibility to metabolic syndrome.

**9.** A screening method for identifying a compound which interacts with a polypeptide that is predominately expressed in visceral adipose tissue, wherein said polypeptide comprises SEQ ID No. 2, said method comprising:

contacting said polypeptide with a compound or a plurality of compounds under conditions which allow interaction of said compound with said polypeptide; and
detecting the interaction between said compound or plurality of compounds with said polypeptide.

**10.** An ex vivo screening method for identifying a compound which is an agonist or an antagonist of a polypeptide that is predominately expressed in visceral adipose tissue, wherein said polypeptide comprises SEQ ID No. 2, said method comprising:

contacting said polypeptide with a compound under conditions which allow interaction of said compound with said polypeptide;
determining a first level of activity of said polypeptide;
determining a second level of activity of said polypeptide expressed in a host which has not been contacted with said compound; and
quantitatively relating the first level of activity with the second level of activity, wherein when said first level of activity is less than said second level of activity, said compound is identified as an antagonist of said polypeptide.

**11.** An ex vivo screening method for identifying a compound which is an inhibitor of the expression of a polypeptide that is predominately expressed in visceral adipose tissue, wherein said polypeptide comprises SEQ ID No. 2, said method comprising:

contacting a host which expresses said polypeptide with a compound;
determining a first expression level or activity of said polypeptide;
determining a second expression level or activity of said polypeptide in a host which has not been contacted with said compound; and
quantitatively relating the first expression level or activity with the second expression level or activity, wherein when said first expression level or activity is less than said second expression level or activity, said compound is identified as an inhibitor of the expression of said polypeptide.

**12.** A method of correlating protein levels in a mammal with a diagnosis of the level of visceral adipose tissue, comprising:

determining the level of a protein comprising SEQ. ID. No. 2 in a biological sample of said mammal; and
generating an index number, Y, which indicates a base level of visceral adipose tissue.

**13.** The method according to claim 12, further comprising comparing index number, Y, to index numbers of subjects known to have specified levels of visceral adipose tissue.

**14.** The method of claim 12 or 13, further comprising monitoring said index number, Y, over time, to determine the progression of the level of visceral adipose tissue, thereby predicting a susceptibility to developing metabolic syndrome.

**15.** Use of a nucleic acid comprising SEQ ID No. 1 as a marker in the diagnosis of visceral adipose tissue accumulation.

**16.** Use of a polypeptide comprising SEQ ID No. 2 as a marker for the diagnosis of visceral adipose tissue accumulation.

## Patentansprüche

**1.** . Verfahren zur Messung der Anteile an viszeralem Fettgewebe, umfassend: das Detektieren oder Messen der Konzentration eines Polypeptidmarkers in einer biologischen Probe, wobei der Polypeptidmarker ein Polypeptid, umfassend SEQ ID Nr. 2, ist.

**2.** . Verfahren nach Anspruch 1, wobei die biologische Probe aus der Gruppe, bestehend aus Serum, Plasma und Zellen von viszeralem Fettgewebe, stammt.

**3.** . Verfahren nach Anspruch 1 oder 2, wobei die Konzentration des Polypeptidmarkers bei einem Individuum, das bekanntermaßen erhöhte Anteile an viszeralem Fettgewebe hat, mit den Expressionsniveaus desselben Polypeptidmarkers bei einem Individuum, das bekanntermaßen niedrige oder normale Anteile an viszeralem Fettgewebe hat, verglichen wird.

**4.** . Verfahren nach einem der Ansprüche 1 bis 3, wobei ein Anstieg der Konzentration des Polypeptidmarkers mit der Zeit für das Stoffwechselsyndrom oder die Anfälligkeit für das Stoffwechselsyndrom indikativ ist.

**5.** . Verfahren zum Messen des Anteils an viszeralem Fettgewebe bei einem Patienten, umfassend: das Detektieren oder Messen der Konzentration eines Nukleinsäuremarkers in einer biologischen Probe, wobei der Nukleinsäuremarker ein Nukleinsäuremolekül mit SEQ ID Nr. 1 umfaßt.

**6.** . Verfahren nach Anspruch 5, wobei der Nukleinsäuremarker RNA ist.

**7.** . Verfahren nach Anspruch 5 oder 6, wobei das Expressionsniveau des Nukleinsäuremarkers bei einem Individuum, das bekanntermaßen erhöhte Anteile an viszeralem Fettgewebe hat, mit den Expressionsniveaus desselben Nukleinsäuremarkers bei einem Individuum, das beganntermaßen niedrige oder normale Anteile an viszeralem Fettgewebe hat, verglichen wird.

**8.** . Verfahren nach einem der Ansprüche 5 bis 7, wobei ein Anstieg des Expressionsniveaus des Nukleinsäuremarkers mit der Zeit für das Stoffwechselsyndrom oder die Anfälligkeit für das Stoffwechselsyndrom indikativ ist.

**9.** . Screeningverfahren zur Identifikation einer Verbindung, die mit einem Polypeptid, das vorwiegend in viszeralem Fettgewebe exprimiert wird, interagiert, wobei das Polypeptid SEQ ID Nr. 2 umfaßt, wobei das Verfahren:

das Inkontaktbringen des Polypeptids mit einer Verbindung oder einer Vielzahl von Verbindungen unter Bedingungen, die eine Interaktion der Verbindung mit dem Polypeptid gestatten; und
das Detektieren der Interaktion zwischen der Verbindung oder Vielzahl von Verbindungen mit dem Polypeptid umfaßt.

**10.** . Ex-vivo-Screeningverfahren zur Identifikation einer Verbindung, die ein Agonist oder ein Antagonist eines Polypeptids, das vorwiegend in viszeralem Fettgewebe exprimiert wird, ist, wobei das Polypeptid SEQ ID Nr. 2 umfaßt, wobei das Verfahren:

das Inkontaktbringen des Polypeptids mit einer Verbindung unter Bedingungen, die eine Interaktion der Verbindung mit dem Polypeptid gestatten;
das Bestimmen eines ersten Aktivitätsniveaus des Polypeptids;
das Bestimmen eines zweiten Aktivitätsniveaus des Polypeptids bei einem Wirt, der nicht mit dieser Verbindung kontaktiert wurde; und
das quantitative Inzusammenhangbringen des ersten Aktivitätsniveaus mit dem zweiten Aktivitätsniveau umfaßt, wobei, wenn das erste Aktivitätsniveau geringer als das zweite Aktivitätsniveau ist, die Verbindung als ein Antagonist des Polypeptids identifiziert wird.

**11.** . Ex-vivo-Screeningverfahren zur Identifikation einer Verbindung, welche ein Inhibitor der Expression eines Polypeptids, das vorwiegend in viszeralem Fettgewebe exprimiert wird, ist, wobei das Polypeptid SEQ ID Nr. 2 umfaßt, wobei das Verfahren:

das Inkontaktbringen eines Wirts, welcher das Polypeptid exprimiert, mit einer Verbindung; das Bestimmen eines ersten Expressionsniveaus oder Aktivität des Polypeptids;
das Bestimmen eines zweiten Expressionsniveaus oder Aktivität des Polypeptids bei einem Wirt, der nicht mit dieser Verbindung kontaktiert wurde; und
das quantitative Inzusammenhangbringen des ersten Expressionsniveaus oder Aktivität mit dem zweiten Expressionsniveau oder Aktivität umfaßt, wobei, wenn das erste Expressionsniveau oder Aktivität geringer ist; als das zweite Expressionsniveau oder Aktivität, die Verbindung als ein Inhibitor der Expression des Polypeptids identifiziert wird.

**12.** . Verfahren zur Korrelation von Proteinkonzentrationen bei einem Säuger mit einer Diagnose des Anteils an viszeralem Fettgewebe, umfassend:

das Bestimmen der Konzentration eines Proteins, umfassend SEQ ID Nr. 2, in einer biologischen Probe des Säugers und
das Erzeugen einer Indexzahl Y, die ein Grundniveau an viszeralem Fettgewebe angibt.

**13.** . Verfahren nach Anspruch 12, ferner umfassend das Vergleichen der Indexzahl Y mit den Indexzahlen von Patienten, die bekanntermaßen spezifizierte Anteile an viszeralem Fettgewebe haben.

**14.** . Verfahren nach Anspruch 12 oder 13, ferner umfassend das Überwachen der Indexzahl Y über die Zeit hinweg, um die Progression des Anteils an viszeralem Fettgewebe zu bestimmen, wodurch eine Anfälligkeit für die Entwicklung eines Stoffwechselsyndroms vorhergesagt wird.

**15.** . Verwendung einer Nukleinsäure, umfassend SEQ ID Nr. 1, als Marker für die Diagnose viszeraler Fettgewebeakkumulation.

**16.** . Verwendung eines Polypeptids, umfassend SEQ ID Nr. 2, als Marker für die Diagnose viszeraler Fettgewebeakkumulation.

## Revendications

**1.** Procédé de mesure des taux de tissu adipeux viscéral comprenant : la détection ou la mesure du taux d'un marqueur polypeptidique dans un échantillon biologique, dans lequel ledit marqueur polypeptidique est un polypeptide comprenant SEQ ID NO : 2.

**2.** Procédé selon la revendication 1, dans lequel ledit échantillon biologique est dérivé du groupe constitué de sérum, de plasma et de cellules du tissu adipeux viscéral.

**3.** Procédé selon la revendication 1 ou 2, dans lequel le taux dudit marqueur polypeptidique chez un individu connu pour avoir des taux élevés de tissu adipeux viscéral est comparé aux taux d'expression du même marqueur polypeptidique chez un individu connu pour avoir des taux faibles ou normaux de tissu adipeux viscéral.

**4.** Procédés selon l'une quelconque des revendications 1 à 3, dans lesquels une augmentation du taux dudit marqueur polypeptidique dans le temps est indicative du syndrome métabolique ou de la sensibilité au syndrome métabolique.

**5.** Procédé de mesure du taux de tissu adipeux viscéral chez un sujet comprenant : la détection ou la mesure du taux d'un marqueur d'acide nucléique dans un échantillon biologique dans lequel ledit marqueur d'acide nucléique comprend une molécule d'acide nucléique de SEQ ID NO : 1.

**6.** Procédé selon la revendication 5, dans lequel ledit marqueur d'acide nucléique est de l'ARN.

**7.** Procédé selon la revendication 5 ou 6, dans lequel le taux d'expression dudit marqueur d'acide nucléique chez un individu connu pour avoir des taux élevés de tissu adipeux viscéral est comparé aux taux d'expression du même marqueur d'acide nucléique chez un individu connu pour avoir des taux faibles ou normaux de tissu adipeux viscéral.

**8.** Procédé selon l'une quelconque des revendications 5 à 7, dans lequel une augmentation du taux d'expression dudit marqueur d'acide nucléique dans le temps est indicative du syndrome métabolique ou de la sensibilité au syndrome métabolique.

**9.** Procédé de criblage pour identifier un composé qui interagit avec un polypeptide qui est exprimé de manière prédominante dans le tissu adipeux viscéral, dans lequel ledit polypeptide comprend SEQ ID NO : 2, ledit procédé comprenant :

la mise en contact dudit polypeptide avec un composé ou une pluralité de composés dans des conditions qui permettent l'interaction dudit composé avec ledit polypeptide ; et
la détection de l'interaction entre ledit composé ou ladite pluralité de composés avec ledit polypeptide.

**10.** Procédé de criblage *ex vivo* pour identifier un composé qui est un agoniste ou un antagoniste d'un polypeptide qui est exprimé de manière prédominante dans le tissu adipeux viscéral, dans lequel ledit polypeptide comprend SEQ ID NO : 2, ledit procédé comprenant :

la mise en contact dudit polypeptide avec un composé dans des conditions qui permettent l'interaction dudit composé avec ledit polypeptide ;
la détermination d'un premier taux d'activité dudit polypeptide ;
la détermination d'un second taux d'activité dudit polypeptide exprimé dans un hôte qui n'a pas été mis en contact avec ledit composé ; et
la relation quantitative du premier taux d'activité avec le second taux d'activité, où lorsque le premier taux d'activité est inférieur au dit second taux d'activité, ledit composé est identifié comme un antagoniste dudit polypeptide.

**11.** Procédé de criblage *ex vivo* pour identifier un composé qui est un inhibiteur de l'expression d'un polypeptide qui est exprimé de manière prédominante dans le tissu adipeux viscéral, dans lequel ledit polypeptide comprend SEQ ID NO : 2, ledit procédé comprenant :

la mise en contact d'un hôte qui exprime ledit polypeptide avec un composé ;
la détermination d'un premier taux d'expression ou de l'activité dudit polypeptide ;
la détermination d'un second taux d'expression ou de l'activité dudit polypeptide dans un hôte qui n'a pas été mis en contact avec ledit composé ; et
la relation quantitative du premier taux d'expression ou de l'activité avec le second taux d'expression ou l'activité, où lorsque le premier taux d'expression ou l'activité est inférieur au dit second taux d'expression ou à l'activité, ledit composé est identifié comme un inhibiteur de l'expression dudit polypeptide.

**12.** Procédé de corrélation des taux de protéines chez un mammifère avec un diagnostic du taux de tissu adipeux viscéral, comprenant ;

la détermination du taux d'une protéine comprenant SEQ ID NO : 2 dans un échantillon biologique dudit mammifère ; et
la génération d'un indice numérique, Y, qui indique un taux de base de tissu adipeux viscéral.

**13.** Procédé selon la revendication 12, comprenant en outre la comparaison de l'indice numérique, Y, aux indices numériques de sujets connus pour avoir des taux spécifiés de tissu adipeux viscéral.

**14.** Procédé selon la revendication 12 ou 13, comprenant en outre le contrôle dudit indice numérique, Y, dans le temps, pour déterminer la progression du taux de tissu adipeux viscéral, prédisant de cette manière une sensibilité pour le développement d'un syndrome métabolique.

**15.** Utilisation d'un acide nucléique comprenant SEQ ID NO : 1 comme marqueur dans le diagnostic d'une accumulation de tissu adipeux viscéral.

**16.** Utilisation d'un polypeptide comprenant SEQ ID NO : 2 comme marqueur pour le diagnostic d'une accumulation de tissu viscéral adipeux.

Figure 1

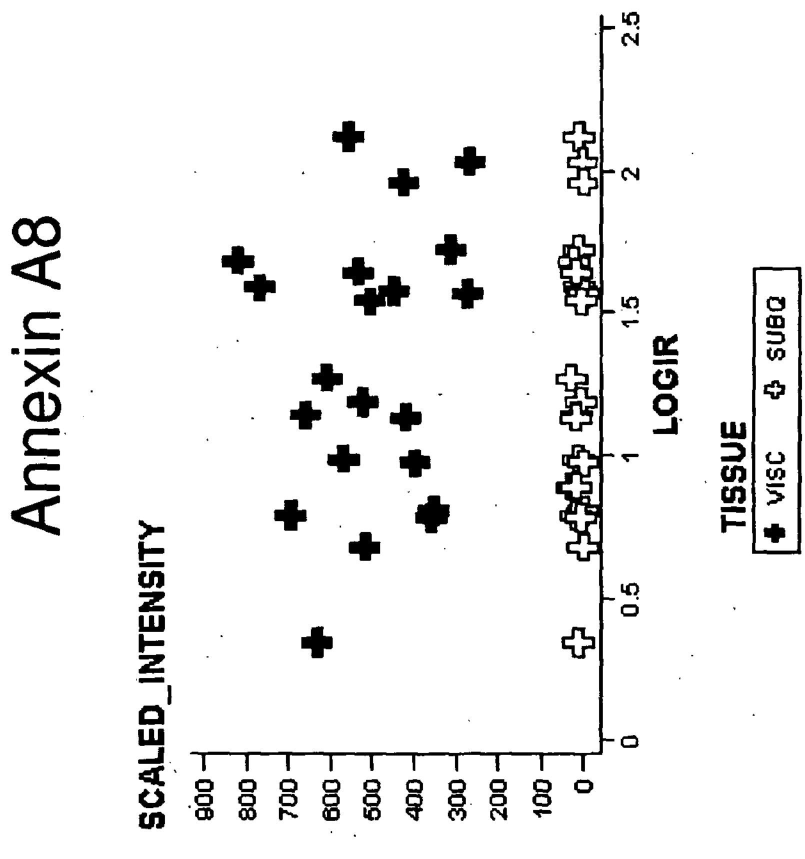
Annexin A8
SCALED_INTENSITY
900
800
700
600
500
400
300
200
100
0
0
0.5
1
1.5
2
2.5
LOGIR
TISSUE
VISC
SUBQ

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9604912 A
- EP 0963376 A
- WO 9825947 A
- WO 0002911 A
- US 6004746 A
- US 5541109 A
- US 5525490 A
- WO 9951741 A
- WO 0017221 A
- WO 0014271 A
- WO 0005410 A


### Non-patent literature cited in the description


- **MARIN, P.** Neuro-endocrine Syndrome. *Neuroendocrine News,* 1996, vol. 21 (3), 2
- **PETER T. KATZMARZYK et al.** *American Journal of Human Biology,* 1999, vol. 11, 225-235
- **BETTINA WEBER-HAMANN et al.** *Psychosomatic Medicine,* 2002, vol. 64, 274-277
- **MARTIN BROCHU et al.** *The Journal of Clinical Endocrinology & Metabolism,* 2000, vol. 85 (7
- **NG.** *Science,* 1999, vol. 283, 2085-2089
- **UBARRETXENA-BELANDIA.** *Biochem.,* 1999, vol. 38, 7398-7405
- **FERNANDEZ.** *Curr. Opin. Chem. Biol.,* 1998, vol. 2, 547-603
- **KASUS-JACOBI.** *Oncogene,* 2000, vol. 19, 2052-2059
- **VIDAL ; LEGRAIN.** *Nucleic Acids Research,* 1999, vol. 27, 919-929
- **NIELSON.** *Science,* 1991, vol. 274, 1497-1500
- **BERRY.** *Biochem. Soc. Trans.,* 1994, vol. 22, 1033-1036
- **WODAK.** *Ann. N. Y. Acad. Sci.,* 1987, vol. 501, 1-13
- **PABO.** *Biochemistry,* 1986, vol. 25, 5987-5991
- **OSTRESH.** *Methods in Enzymology,* 1996, vol. 267, 220-234
- **DORNER.** *Bioorg. Med. Chem.,* 1996, vol. 4, 709-715
- **ROSE.** *Biochemistry,* 1996, vol. 35, 12933-12944
- **RUTENBER.** *Bioorg. Med. Chem.,* 1996, vol. 4, 1545-1558
- **WODICKA L ; DONG H ; MITTMANN M ; HO MH ; LOCKHART DJ.** Genome-wide expression monitoring in Saccharomyces cerevisiae. *Nat Biotechnol,* 1997, vol. 15, 1359-67
- **DRAPER N. ; SMITH H.** Applied Regression Analysis. John Wiley and Sons, 1966
- **SEARLE S. R.** Linear Models. John Wiley and Sons, 1971
- **SOKAL R. R. ; ROHLF F.** J. Biometry. W. H. Freeman and Company, 1969

SEQUENCE LISTING

<110> F. Hoffmann-La Roche Ltd.

<120> SPECIFIC MARKERS FOR METABOLIC SYNDROME

<130> 21742

<160> 2

<170> PatentIn version 3.2

<210> 1
<211> 1940
<212> DNA
<213> Homo sapiens

<220>
<221> Annexin A8
<222> (1)..(1940)
<223> X16662

<400> 1

```
aggcctgctc actcctcagc tgcaggagcc agacgtgtgg agtcccagca gaggccaacc    60
tgtgtctctt catctccgtg agaaaggtgc ccccgaagtg aaagagatgg cctggtggaa   120
agcctggatt gaacaggagg gtgtcacagt gaagagcagc tcccacttca acccagaccc   180
tgatgcagag accctctaca aagccatgaa ggggatcggg accaacgagc aggctatcat   240
cgatgtgctc accaagagaa gcaacacgca gcggcagcag atcgccaagt ccttcaaggc   300
tcagttcggc aaggacctca ctgagacctt gaagtctgag ctcagtggca agtttgagag   360
```

gctcattgtg gcccttatgt atccgccata cagatacgaa gccaaggagc tgcatgacgc 420
catgaagggc ttaggaacca aggagggtgt catcattgag atcctggcct ctcggaccaa 480
gaaccagctg cgggagataa tgaaggcgta tgaggaagac tatgggtcca gcctggagga 540
ggacatccaa gcagacacaa gtggctacct ggagaggatc ctggtgtgcc tcctgcaggg 600
cagcagggat gatgtgagca gctttgtgga cccggcactg gccctccaag acgcacagga 660
tctgtatgcg gcaggcgaga agattcgtgg gactgatgag atgaaattca tcaccatcct 720
gtgcacgcgc agtgccactc acctgctgag agtgtttgaa gagtatgaga aaattgccaa 780
caagagcatt gaggacagca tcaagagtga gacccatggc tcactggagg aggccatgct 840
cactgtggtg aaatgcaccc aaaacctcca cagctacttt gcagagagac tctactatgc 900
catgaaggga gcagggacgc gtgatgggac cctgataaga aacatcgttt caaggagcga 960
gattgactta aatcttatca aatgtcactt caagaagatg tacggcaaga ccctcagcag 1020
catgatcatg gaagacacca gcggcgacta caagaacgcc ctgctgagcc tggtgggcag 1080
cgacccctga ggcacagaag aacaagagca aagaccatga agccagagtc tccaggactc 1140
ctcactcaac ctcggccatg gacgcaggtt gggtgtgagg ggggtcccag cctttcggtc 1200
ttctatttcc ctatttccag tgctttccag ccgggtttct gacccagagg tggaaccggc 1260
ctggactcct cttcccaact tcctccaggt catttcccag tgtgagcaca atgccaacct 1320
tagtgtttct ccagccagac agatgcctca gcatgaaggg cttggggact tgtggatcat 1380
tccttcctcc ctgcaggagc ttcccaagct ggtcacagag tctcctgggc acaggttata 1440
cagaccccag ccccattccc atctactgaa acagggtctc cacaagaggg gccagggaat 1500
atgggttttt aacaagcgtc ttacaaaaca cttctctatc atgcagccgg agagctggct 1560
gggagccctt ttgttttaga acacacatcc ttcagcagct gagaaatgaa cacgaatcca 1620
tcccaaccga gatgccatta acattcatct aaaaatgtta ggctctaaat ggacgaaaaa 1680

ttctctcgcc atcttaataa caaaataaac tacaaattcc tgacccaagg acactgtgtt 1740

ataagaggcg tgggctcccc tggtggctga ccaggtcagc tgccctggcc ttgcacccct 1800

ctgcatgcag cacagaaggg tgtgaccatg ccctcagcac cactcttgtc cccactgaac 1860

ggcaactgag actgggtacc tggagattct gaagtgcctt tgctgtggtt ttcaaaataa 1920

taaagatttg tattcaactc 1940

<210> 2
<211> 327
<212> PRT
<213> Homo sapiens

<220>
<221> Annexin A8
<222> (1)..(327)
<223> NP001621

<400> 2

Met Ala Trp Trp Lys Ala Trp Ile Glu Gln Glu Gly Val Thr Val Lys
1 5 10 15

Ser Ser Ser His Phe Asn Pro Asp Pro Asp Ala Glu Thr Leu Tyr Lys
20 25 30

Ala Met Lys Gly Ile Gly Thr Asn Glu Gln Ala Ile Ile Asp Val Leu

35 40 45

Thr Lys Arg Ser Asn Thr Gln Arg Gln Gln Ile Ala Lys Ser Phe Lys

50 55 60

Ala Gln Phe Gly Lys Asp Leu Thr Glu Thr Leu Lys Ser Glu Leu Ser

65 70 75 80

Gly Lys Phe Glu Arg Leu Ile Val Ala Leu Met Tyr Pro Pro Tyr Arg

85 90 95

Tyr Glu Ala Lys Glu Leu His Asp Ala Met Lys Gly Leu Gly Thr Lys

100 105 110

Glu Gly Val Ile Ile Glu Ile Leu Ala Ser Arg Thr Lys Asn Gln Leu

115 120 125

Arg Glu Ile Met Lys Ala Tyr Glu Glu Asp Tyr Gly Ser Ser Leu Glu

130 135 140

Glu Asp Ile Gln Ala Asp Thr Ser Gly Tyr Leu Glu Arg Ile Leu Val

145 150 155 160

Cys Leu Leu Gln Gly Ser Arg Asp Asp Val Ser Ser Phe Val Asp Pro

165 170 175

Ala Leu Ala Leu Gln Asp Ala Gln Asp Leu Tyr Ala Ala Gly Glu Lys

180 185 190

Ile Arg Gly Thr Asp Glu Met Lys Phe Ile Thr Ile Leu Cys Thr Arg

195 200 205

Ser Ala Thr His Leu Leu Arg Val Phe Glu Glu Tyr Glu Lys Ile Ala

```
    210                 215                 220
Asn Lys Ser Ile Glu Asp Ser Ile Lys Ser Glu Thr His Gly Ser Leu
225                 230                 235                 240
Glu Glu Ala Met Leu Thr Val Val Lys Cys Thr Gln Asn Leu His Ser
                245                 250                 255
Tyr Phe Ala Glu Arg Leu Tyr Tyr Ala Met Lys Gly Ala Gly Thr Arg
            260                 265                 270

Asp Gly Thr Leu Ile Arg Asn Ile Val Ser Arg Ser Glu Ile Asp Leu
        275                 280                 285
Asn Leu Ile Lys Cys His Phe Lys Lys Met Tyr Gly Lys Thr Leu Ser
    290                 295                 300
Ser Met Ile Met Glu Asp Thr Ser Gly Asp Tyr Lys Asn Ala Leu Leu
305                 310                 315                 320
Ser Leu Val Gly Ser Asp Pro
                325
```